# NEUE EUROPÄISCHE PATENTSCHRIFT

(11) **EP 3 659 594 B2**
(45) Veröffentlichungstag und Bekanntmachung des Hinweises auf die Entscheidung über den Einspruch: **29.04.2026**
(45) Hinweis auf die Patenterteilung: 18.11.2020
(21) Anmeldenummer: 18209583.6
(22) Anmeldetag: 30.11.2018
(51) Int. Cl.: A61K 31/216, A61M 5/178, A61K 9/08

(54) **EINE STERILE LÖSUNG MIT EINEM MEDIZINISCHEN WIRKSTOFF ENTHALTENDE APPLIKATIONSSPRITZE UND VERFAHREN FÜR DEREN BEREITSTELLUNG**
A STERILE SOLUTION WITH AN APPLICATION INJECTOR COMPRISING A MEDICAL AGENT AND METHOD FOR PRODUCING SAME
SERINGUE D'ADMINISTRATION DE SOLUTION STÉRILE COMPORTANT UNE SUBSTANCE ACTIVE MÉDICALE ET SON PROCÉDÉ D'UTILISATION

(43) Veröffentlichungstag der Anmeldung: 03.06.2020
(73) Patentinhaber: Stegemann, Klaus, 21035 Hamburg (DE)
(72) Erfinder: Stegemann, Klaus, 21035 Hamburg (DE)
(74) Vertreter: Raffay & Fleck

(56) Entgegenhaltungen:
- WO-A1-2012/166045
- WO-A1-2013/184270
- WO-A1-2014/131742
- WO-A1-2014/131742
- WO-A1-2016/187078
- WO-A1-2017/085253
- JP-A- 2003 260 143
- US-A1- 2016 058 715
- WALTER SCHIESS: "Selection of materials and surfaces for small volume parenteral primary packaging", 12 June 2007 (2007-06-12), XP002791594, Retrieved from the Internet <URL:http://www.gampitalia.it/documenti/research-development-and-innovation-in-technology-for-pharmaceutical-processing/4-walter-schiess.pdf/view>

## Beschreibung

Die vorliegende Erfindung betrifft eine eine sterile Lösung mit einem medizinischen Wirkstoff enthaltende Applikationsspritze, insbesondere für das Einbringen der Lösung über einen Harnwegs- und/oder Blasenkatheter in die Blase eines Patienten. Sie betrifft ferner ein Verfahren zum Bereitstellen einer solchen eine sterile Lösung mit einem medizinischen Wirkstoff enthaltenden Applikationsspritze.

Es ist bekannt, einen oder mehrere medizinische(n) Wirkstoff(e) enthaltende Lösungen in Applikationsspritzen abgefüllt für medizinische Behandlungen an Ärzte oder auch an Patienten für die Selbstmedikation abzugeben.

So werden bspw. Parasympatholytika, wie etwa Oxybutynin-Hydrochlorid, für die Behandlung von überaktiver Blase eingesetzt. Insbesondere werden solche Parasympatholytika, wie etwa Oxybutynin-Hydrochlorid, zum Relaxieren der Blase bei Patienten eingesetzt, die ihre Blase nicht mehr auf natürlichem Wege entleeren können, auf die Entleerung der Blase über einen in die Harnröhre eingeführten Katheter angewiesen sind. Zu solchen Patienten zählen zum Beispiel und insbesondere Patienten mit Querschnittslähmung.

Parasympatholytika, wie eben Oxybutynin-Hydrochlorid-haltige Medikamente, werden dabei insbesondere oral verabreicht. Das Problem bei dieser Darreichungsform besteht in einer durch die verzögerte Aufnahme über den Magen-Darmtrakt verzögerten Wirkung und weiterhin in den bei dieser Darreichungsform zu beobachtenden Nebenwirkungen. Zu diesen Nebenwirkungen gehören Mundtrockenheit, Verstopfung, Sehstörungen, Müdigkeit und Schwindel. In besonders schweren Fällen kann es auch zu einem durch das Parasympatholytikum, wie z.B. Oxybutynin-Hydrochlorid, verursachten Delirium kommen.

Insoweit sind auch bereits weitere Darreichungsmöglichkeiten entwickelt worden, die keine orale Gabe, sondern eine transdermale Verabreichung des Wirkstoffs vorsehen, zum Beispiel mit transdermalen Pflastern oder Gelen. Diese Art der Verabreichung des Wirkstoffs kann zwar Nebenwirkungen reduzieren, führt jedoch weiterhin zu einer verzögerten Wirkung des Medikaments.

Es gab weiterhin auch bereits Versuche und praktische Umsetzungen, bei denen ein Parasympatholytikum, z.B. Oxybutynin-Hydrochlorid, enthaltendes Medikament in Form einer Lösung unmittelbar in die Blase instilliert wird, insbesondere nach einem Entleeren der Blase über einen Katheter durch den für die Blasenentleerung bereits gesetzten Katheter. Diese Darreichungsform hat den großen Vorteil, dass nicht nur die Nebenwirkungen deutlich verringert sind, sondern vor allem die Wirkung des Medikaments sehr zeitnah, weitgehend unmittelbar, erfolgt, da es direkt im Bereich der Blase verabreicht wird und kein Transport über den Blutkreislauf erforderlich ist. WO 2014/131742 beschreibt ein Kit bestehend aus einem Katheter und einer Kolbenspritze enthaltend ein Medikament.

Bei den bisherigen Anwendungen dieser Art, bei denen eine ein Parasympatholytikum enthaltende, z.B. eine Oxybutynin-Hydrochlorid-haltige, Lösung in einer Applikationsspritze dargereicht wird, die der Anwender selbstständig nach der Entleerung der Blase über den gesetzten Blasenkatheter verabreichen kann, wurden einfache Applikationsspritzen verwendet, die aus Polyethylen (PE) oder Polypropylen (PP) hergestellt waren und einen Kolben am Spritzenstempel aus einem einfachen Kautschukmaterial aufwiesen. Weiterhin waren diese Applikationsspritzen mit einem eine Auslassöffnung umgebenden Luer-Lock-Anschluss verwendet worden, auf den ein passender Adapterkonus zum Verbinden mit dem jeweils vom Patienten für die Entleerung seiner Blase eingesetzten Katheter aufgeschraubt war.

In aufwändigen Versuchen hat der Erfinder festgestellt, dass diese vorbekannten Lösungen verschiedene Nachteile mit sich bringen. Insbesondere musste der Erfinder feststellen, dass in den bisher verwendeten Applikationsspritzen sich insbesondere dann, wenn diese für eine abschließende Sterilisierung einer Dampfdruck-Behandlung unterzogen und dabei auf die dort üblichen Temperaturen von typischerweise mindestens 120 °C (zum Beispiel 121 °C) erhitzt werden, sich die Konzentration des Wirkstoffs, z.B. von Oxybutynin-Hydrochlorid, in der in der Applikationsspritze enthaltenen Lösung verändert, dass diese Konzentration abnimmt. Diese Abnahme der Wirkstoffkonzentration konnte der Erfinder in weiteren und aufwändigen Versuchen darauf zurückführen, dass das bisher eingesetzte Material der Applikationsspritzen, einerseits des Spritzenkörpers selbst, nämlich das PP und das PE, andererseits des Stopfens, nämlich der gewöhnliche Kautschuk, im Zuge der Dampfdruck- Sterilisierung in der Lösung enthaltenen Wirkstoff, z.B. Oxybutynin-Hydrochlorid, absorbiert und auch nach der Dampfdruck-Behandlung dort gebunden hält.

Diesem Problem zu begegnen und eine eine sterile Oxybutynin-Hydrochlorid-haltige Lösung enthaltende Applikationsspritze bereitzustellen, die in einem Dampfdruck-Verfahrensterilisierbarist, ohne dabei die Konzentration des medizinischen Wirkstoffs in der Lösung zu verändern, hat sich der Erfinder zur Aufgabe gemacht.

Diese Aufgabe wird erfindungsgemäß gelöst durch eine eine sterile Oxybutynin-Hydrochlorid-haltige Lösung enthaltende Applikationsspritze, insbesondere für das Einbringen des Medikaments über einen Harnwegs- und/oder Blasenkatheter in die Blase eines Patienten, und mit einer Applikationsspritze mit einem zylinderförmigen, einseitig durch einen einstückig mit dem Mantel ausgebildeten eine Auslassöffnung aufweisenden Spritzendeckel verschlossenen Mantel aus einem Cycloolefin-Copolymer (COC) und einem Kolben aus einemhalogenierten Isobuten-Isopren-halogenierter (auch als halogenierter Butylkautschuk bezeichnet), bei der die den medizinischen Wirkstoff enthaltenden Lösung in einem von dem zylinderförmigen Mantel mit Spritzendeckel und dem Kolben begrenzten Hohlraum angeordnet ist.

In aufwändigen Versuchen hat der Erfinder nämlich herausgefunden, dass die nun von ihm für die Applikationsspritze, genauer deren Mantel mit Spritzendeckel sowie für den Kolben vorgesehenen Materialien COC und halogenierter Isobuten-Isopren-Kautschuk auch bei solchen Verhältnissen und insbesondere Temperaturen, wie sie für eine abschließende Dampfdruck-Sterilisierungeingesetzt werden, den medizinsichen Wirkstoff Oxybutynin-Hydrochlorid nicht absorbieren, dass jedenfalls nach dem Dampfdruck-stilisieren die Wirkstoffkonzentration in der in der so behandelten Applikationsspritze enthaltenen den medizinischen Wirkstoff enthaltenden Lösung nicht verändert ist.

Dadurch kann nun erstmals ein aufgrund der abschließenden Dampfdruck-Sterilisierung den geforderten Vorgaben an die Sterilität entsprechendes fertiges Produkt angeboten werden, welches die sterile Lösung mit dem medizinischen Wirkstoff in der eingefüllten Konzentration enthält und somit mit der gewünschten Wirkung vom Behandler oder einem betroffenen Patienten in einer Selbstbehandlung eingesetzt werden kann.

Die sterile Lösung enthält als medizinischen Wirkstoff Oxybutynin-Hydrochlorid.

In der erfindungsgemäßen Applikationsspritze ist die Auslassöffnung in einem einstückig mit dem Mantel und dem Spritzendeckel ausgebildeten Stufenkonus mit wenigstens zwei, vorzugweise wenigstens drei, zylinderförmigen Abschnitt mit unterschiedlichen, zu einer Spitze des Stufenkonus hin kleiner werdenden Durchmessern ausgebildet. Diese Ausgestaltung erlaubt z.B. ein Koppeln der Applikationsspritze mit unterschiedlich gestalteten, unterschiedliche Anschlusskonusse aufweisenden Kathetern, z.B. solchen für die Blasenentleerung. Die einstückige Ausbildung des Stufenkonus mit dem Mantel und dem Spritzendeckel ergibt des Weiteren eine deutliche Verbesserung gegenüber den vorbekannten Bauformen mit über eine Luer-Lock-Verbindung an der Applikationsspritze festgelegten Adapterkonussen. Denn diese, auch dies hat der Erfinder in Versuchen festgestellt, halten bei einer abschließenden Dampfdruck-Sterilisierung der befüllten und mit dem Adapterkonus verbundenen Applikationsspritze nicht ausreichend dicht, sodass hier ebenfalls eine Veränderung der den medizinischen Wirkstoff enthaltenden Lösung, die in der Applikationsspritze angeordnet ist, die Folge sein kann. Um mit dem in der vorteilhaften Weiterbildung erfindungsgemäß einstückig mit dem Mantel und dem Spritzendeckel ausgebildeten Stufenkonus möglichst flexibel eine große Zahl von Kathetern, z.B. Kathetern für die Blasenentleerung, konnektieren zu können, kann der Stufenkonus eine große Vielzahl von zylinderförmigen Abschnitten mit unterschiedlichen, zu der Spitze des Stufenkonus hin kleiner werdenden Durchmessern aufweisen, zum Beispiel 10 bis 15 solche zylinderförmigen Abschnitte, insbesondere beispielsweise 12.

Das Material des Kolbens kann insbesondere autoklavierbarer Brombutylkautschuk sein.

Gemäß der vorliegenden Erfindung ist der in der sterilen Lösung enthaltene medizinische Wirkstoff ein Parasympatholytikum, nämlich Oxybutynin-Hydrochlorid. Insbesondere kann dieser Wirkstoff für die Anwendung zum Relaxieren der Blase eines Patienten ausgewählt und in der sterilen Lösung entsprechend dosiert sein.

Für eine mit der sterilen Lösung, mit der die Applikationsspritze befüllt ist, durchzuführende Medikation für eine Relaxation einer überaktiven Blase haben sich sterile Lösungen mit einem Anteil des genannten Wirkstoffs von 0,01 bis 1,0 Gew.-%, vorzugsweise von 0,05 bis 0,5 Gew.-%, insbesondere von 0,08 bis 0,12 Gew.-%, mit Vorteil von 0,1 Gew.-% als geeignet erwiesen.

Mit Vorteil kann die sterile Lösung, die in dem Hohlraum der Applikationsspritze angeordnet ist, eine Oxybutynin-Hydrochlorid-haltige NaCl-Lösung sein. Hierbei kann es sich insbesondere um eine Oxybutynin-Hydrochlorid-haltige, isotonische NaCl-Lösung handeln.

Eine für eine einmalige Medikation geeignete Menge, ein geeignetes Volumen, der sterilen, Oxybutynin-Hydrochlorid enthaltenden Lösung kann insbesondere von 1 bis 50 ml, vorzuweisen von 5 bis 20 ml, insbesondere von 8 bis 12 ml, kann mit besonderem Vorteil 10 ml, betragen.

Mit Vorteil ist die mit der sterilen, den medizinischen Wirkstoff enthaltenden Lösung befüllte Applikationsspritze in einem Auslieferungszustand an ihrer Auslassöffnung durch ein Verschlusselement, insbesondere einen Verschlussstopfen oder eine Verschlusskappe, verschlossen, wobei dieses Verschlusselement aus einem, insbesondere halogenierten, Isobuten-Isopren-Kautschuk besteht. Es kann insbesondere aus einem autoklavierbaren Brombutylkautschuk gebildet sein. Das Verschlusselement verhindert ein verfrühtes Austreten der den medizinischen Wirkstoff enthaltenden, sterilen Lösung aus dem Hohlraum der Applikationsspritze und gewährleistet, dass der Bereich um die Auslassöffnung, der von dem Verschlussstopfen oder der Verschlusskappe überdeckt ist, vor mikrobieller Kontamination geschützt wird, und so nach einem Sterilisierungsvorgang bis zu einem Verpacken der Applikationsspritze in z.B. einer Blisterverpackung, steril bleibt. Die bezeichnete Materialwahl für den Verschlussstopfen bzw. die Verschlusskappe führt wiederum erneut dazu, dass auch das Verschlusselement in einem abschließenden Dampfdruck-Sterilisationsprozess keinen Wirkstoff.

Die erfindungsgemäße mit der den medizinischen Wirkstoff enthaltenden Lösung befüllte Applikationsspritze wird insbesondere im Prozess endsterilisiert und anschließend, z.B. in einer Blisterverpackung, verpackt, sodass, wenn der Nutzer die Applikationsspritze unmittelbar vor der Anwendung der Verpackung entnimmt,der Spritzeninhalt dann steril und keimfrei ist und damit verhindert wird, dass z.B. bei einer Behandlung der Blase Keime in den Harnleiter- und Blasentrakt eingeschleppt werden.

Mit der Erfindung wird weiterhin auch ein Verfahren zum Bereitstellen einer eine sterile Lösung mit einem medizinischen Wirkstoff enthaltenden Applikationsspritze, insbesondere für das Einbringen der sterilen Lösung über einen Harnwegs- und/oder Blasenkatheter in die Blase eines Patienten, angegeben, welche Applikationsspritze wie vorstehend beschrieben gebildet ist. Das Verfahren umfasst dabei folgende Schritte:
Es wird eine sterile, einen zylinderförmigen, einseitig durch einen einstückig mit dem Material ausgebildeten, eine Auslassöffnung aufweisenden Spritzendeckel verschlossenen Mantel aus einem Cycloolefin-Copolymer (COC) und einen Kolben aus einem halogenierten Isobuten-Isopren-Kautschuk aufweisende Applikationsspritze bereitgestellt. Es wird weiterhin eine den medizinischen Wirkstoff Oxybutynin-Hydrochlorid enthaltende Lösung bereitgestellt. Diese den medizinischen Wirkstoff enthaltende Lösung kann insbesondere entsprechend den vorstehenden, bei der Beschreibung der Applikationsspritze gemachten Angaben gebildet sein, also mit einer, insbesondere isotonischen, NaCl-Lösung und/oder in den vorstehend angegebenen Konzentrationen des Oxybutynin-Hydrochlorid als Wirkstoff. Ein von dem zylinderförmigen Mantel mit Spritzendeckel und von dem Kolben begrenzter Hohlraum der Applikationsspritze wird mit der wirkstoffhaltigen Lösung befüllt. Dies kann insbesondere ein Befüllen des Hohlraums mit einem wie vorstehend bei der Beschreibung der Applikationsspritze angegebenen Volumen der Lösung sein. Die Auslassöffnung der Applikationsspritze wird mit einem Verschlusselement aus einem, insbesondere halogenierten, Isobuten-Isopren-Kautschuk, z.B. einem autoklavierbaren Brombutylkautschuk, verschlossen. Die so gebildete Einheit, also die mit der wirkstoffhaltigen Lösung befüllte und an ihrer Auslassöffnung verschlossene Applikationsspritze, wird abschließend in einem Autoklav Dampfdruck-sterilisiert.

Im Anschluss an das Dampfdruck-Sterilisieren kann die mit der dann sterilisierten wirkstoffhaltigen Lösung befüllte, mit dem Verschlusselement verschlossene Applikationsspritze verpackt werden für eine Auslieferung, Lagerung und den Transport bis zum Patienten und gegebenenfalls einer Aufbewahrung dort.

Insbesondere kann eine wie vorstehend beschriebene Applikationsspritze mit einer in einem einstückig mit dem Mantel und dem Spritzendeckel ausgebildeten Stufenkonus mit wenigstens zwei, vorzuweisen wenigstens drei, zylinderförmigen Abschnitten mit unterschiedlichen, zu einer Spitze des Stufenkonus hin kleiner werdenden Durchmessern ausgebildeten Auslassöffnung bereitgestellt werden. Der Stufenkonus kann dabei wiederum insbesondere in einer solchen Weise gestaltet sein, wie vorstehend anhand der Beschreibung der Applikationsspritze dargelegt und erläutert.

Das Dampfdruck-Sterilisieren in dem Autoklav kann für wenigstens 10 Minuten, insbesondere wenigstens 15 Minuten, insbesondere für 20 Minuten durchgeführt werden und erfolgt vorzugsweise bei wenigstens 115°C, insbesondere wenigstens 120°C, insbesondere bei genau 121 °C.

Weitere Vorteile und Merkmale der Erfindung ergeben sich aus der nachfolgenden Beschreibung eines Ausführungsbeispiels anhand der beiliegenden Figuren. Dabei zeigen:

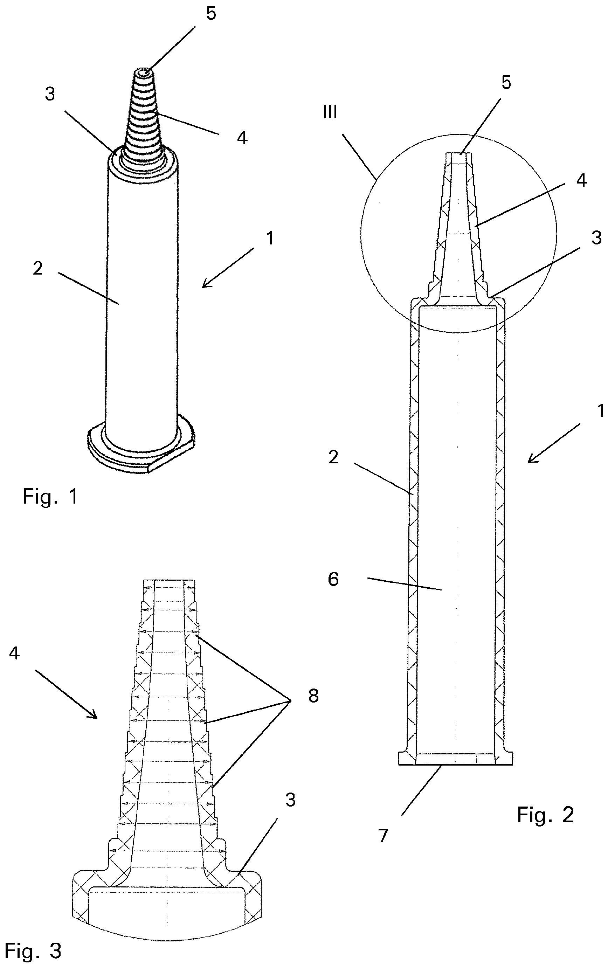

- Fig. 1: eine dreidimensionale Ansicht einer Applikationsspritze gemäß der Erfindung (ohne den Spritzenstempel mit Kolben);
- Fig. 2: eine Schnittdarstellung der in Fig. 1 gezeigten Applikationsspritze, (wiederum ohne den Spritzenstempel mit Kolben); und
- Fig. 3: eine vergrößerte Ansicht des in der Fig. 2 mit III bezeichneten Bereichs.

In den Figuren ist in Prinzipdarstellungen ein mögliches Ausführungsbeispiel einer erfindungsgemäßen Applikationsspritze gezeigt. Dabei sind die Figuren nicht maßstabsgerecht und enthalten auch nicht alle für die Gestaltung der Applikationsspritze erforderlichen Elemente dargestellt. Insbesondere ist in den Figuren ein Spritzenstempel mit dem daran angeordneten Kolben nicht gezeigt, wie er zu der Applikationsspritze gehört. Ein solcher Spritzenstempel ist aber in seiner Formgebung dem Fachmann hinreichend bekannt, sodass auf die bekannten Gestaltungen verwiesen werden kann. Das Besondere an der erfindungsgemäßen Applikationsspritze ist dabei auch nicht die Formgebung, sondern vielmehr das Material des Kolbens.

Eine Applikationsspritze (dargestellt, wie erwähnt ohne den Spritzenstempel mit Kolben) ist in den Figuren allgemein mit dem Bezugszeichen 1 bezeichnet. Diese Applikationsspritze 1 beinhaltet einen Mantel 2, mit dem ein Spritzendeckel 3 einstückig ausgebildet ist. Weiterhin einstückig an dem Spritzendeckel 3 angeformt ist in dem gezeigten Ausführungsbeispiel ein Stufenkonus 4, der sich aus einer Vielzahl von aufeinander gesetzten zylinderförmigen Abschnitten 8 mit ausgehend von dem Spritzendeckel 3 bis hin zu einer Spitze des Stufenkonus 4 jeweils kleiner werdenden Durchmessern aus der Lösung absorbiert und damit die Wirkstoffkonzentration in der sterilen Lösung nicht verändert wird. zusammensetzt. In dem gezeigten Ausführungsbeispiel sind dies insgesamt zwölf solche Abschnitte 8, die über einen Hals in den Spritzendeckel 3 einstückig übergehen.

Dieser einstückig an dem Spritzendeckel 3 angeformte und somit mit dem Mantel 2 verbundene Stufenkonus 4 erlaubt ein Konnektieren der Applikationsspritze 1 mit konischen Anschlussöffnungen von Kathetern, z.B. solchen für das Entleeren von Blasen, mit ganz unterschiedlicher Form.

Das Besondere an der erfindungsgemäßen Applikationsspritze 1 ist nun, dass diese einerseits mit dem Mantel 2, dem Spritzendeckel 3 und dem Stufenkonus 4 einstückig und aus einem besonderen Material, nämlich einem Cycloolefin-Copolymer (COC) gefertigt, z.B. spritzgegossen, ist und einen (in den Figuren nicht gezeigten, in üblicher Weise in den Mantel 2 von einem hinteren Ende 7 her eingeführte) Kolben aus einem besonderen Kautschuk, nämlich einem halogenierten Isobuten-Isopren-Kautschuk, z.B. einem autoklavierbaren Brombutylkautschuk, aufweist. Weiterhin besonders ist, dass diese Applikationsspritze 1 in einem in dem Mantel 2 ausgebildeten und durch den Mantel 2, den Spritzendeckel 3 und den nicht näher dargestellten Kolben begrenzten Hohlraum 6 eine sterile,

Oxybutynin-Hydrochlorid enthaltende Lösung, insbesondere einer Oxybutynin-Hydrochlorid-haltigen NaCl-Lösung, insbesondere einer Oxybutynin-Hydrochlorid-haltigen, isotonischen NaCl-Lösung gefüllt ist. Im Zuge der Bereitung ist die erfindungsgemäße Applikationsspritze 1, deren Auslassöffnung 5 insbesondere zuvor durch eine auf den Stufenkonus 4 aufgesetzte, aus einem, insbesondere halogenierten, Isobuten-Isopren-Kautschuk, vorzugsweise aus autoklavierbarem Brombutylkautschuk, bestehende Kappe, verschlossen worden ist, insbesondere in einem abschließenden Schritt sterilisiert worden, insbesondere in einer Dampfdruck-Sterilisierung in einem Autoklav, zum Beispiel für 20 Minuten bei 121 °C. Die besondere Materialwahl für das Material des Mantels 2 mit einstückig angeformtem Spritzendeckel 3 und Stufenkonus 4 und auch für das Material des Kolbens (sowie des optional verwendbaren Verschlusses in Form einer Verschlusskappe) ermöglicht, dass bei diesem abschließenden Sterilisierungs-Vorgang keine Veränderung des in dem Hohlraum 6 enthaltenen Wirkstoffs, insbesondere hinsichtlich seiner Konzentration in der sterilen Lösung erfolgt. Die gewählten Materialien nehmen auch bei den während der Dampfdruck-Sterilisierung eingestellten Temperaturen diesen Wirkstoff, Oxybutynin-Hydrochlorid, aus der Lösung nicht auf und absorbieren ihn nicht, sodass keine, bei bekannten Applikationsspritzen aus dem Stand der Technik zu beobachtende Veränderung der Wirkstoffkonzentration des Wirkstoffs in der Lösung eintritt.

Die Konzentration einer Oxybutynin-Hydrochlorid enthattenden sterilen Lösung in dem Hohlraum 6 kann insbesondere zwischen 0,01 Gewichtsprozent und 1,0 Gew.-% betragen. In dem hier beschriebenen Ausführungsbeispiel beträgt sie vorzugsweise 0,1 Gew.-%. Das Volumen des in dem Hohlraum 6 aufgenommenen sterilen Lösung kann insbesondere zwischen 1 ml und 50 ml betragen, liegt in dem gezeigten Ausflugsbeispiel vorzugsweise bei 10 ml.

Der Vorteil, dass der Stufenkonus 4 einstückig mit dem Spritzendeckel 3 ausgebildet ist, besteht darin, dass an dieser Stelle eine bei bekannten Applikationsspritzen aus dem Stand der Technik zu beobachtende Leckage nicht auftreten kann, sodass auch hier in dem abschließenden Dampfdruck-Sterilisationsschritt keine Beeinflussung der in dem Hohlraum 6 befindlichen Lösung, insbesondere des darin gelösten medizinischen Wirkstoffs in Form von Oxybutynin-Hydrochlorid stattfinden kann.

Aus der vorstehenden Beschreibung des Ausführungsbeispiels ist noch einmal klar geworden, welche besonderen Vorteile die erfindungsgemäße Ausgestaltung mit sich bringt, indem diese insbesondere eine abschließende Sterilisierung der den medizinischen Wirkstoff enthaltenden Lösung befüllten Applikationsspritze 1 in einem Dampfdruck-Sterilisierungsschritt in einem Autoklavermöglicht, ohne dass dies zu einer Beeinträchtigung der in der Applikationsspritze 1 eingefüllten Lösung und des darin gelösten Wirkstoffs führen würde.

### Bezugszeichenliste

- 1: Applikationsspritze
- 2: Mantel
- 3: Spritzendeckel
- 4: Stufenkonus
- 5: Auslassöffnung
- 6: Hohlraum
- 7: hinteres Ende
- 8: zylinderförmiger Abschnitt

## Patentansprüche

1. Eine sterile Lösung mit einem medizinischen Wirkstoff enthaltende Applikationsspritze (1) für das Einbringen der wirkstoffhaltigen Lösung über einen Harnwegs- und/oder Blasenkatheter in die Blase eines Patienten,
wobei
• die Applikationsspritze (1) einen zylinderförmigen Mantel (2) aufweist, der durch einen einstückig mit dem Mantel (2) ausgebildeten, eine Auslassöffnung (5) aufweisenden Spritzendeckel (3) verschlossen und aus einem Cycloolefin-Copolymer (COC) gebildet ist, und einen Kolben aus einem halogenierten Isobuten-Isopren-Kautschuk aufweist und
• die sterile, den medizinischen Wirkstoff enthaltende Lösung in einem von dem zylinderförmigen Mantel (2) mit Spritzendeckel (3) und dem Kolben begrenzten Hohlraum (6) angeordnet ist,
**dadurch gekennzeichnet, dass** die Auslassöffnung (5) in einem einstückig mit dem Mantel (2) und dem Spritzendeckel (3) ausgebildeten Stufenkonus (4) mit wenigstens zwei, vorzugsweise wenigstens drei, zylinderförmigen Abschnitten (8) mit unterschiedlichen, zu einer Spitze des Stufenkonus (4) hin kleiner werdenden Durchmessern ausgebildet ist
und dass als medizinscher Wirkstoff Oxybutynin-Hydrochlorid in der sterilen Lösung enthalten ist.

2. Applikationsspritze (1) nach Anspruch 1, **dadurch gekennzeichnet, dass** das Oxybutynin-Hydrochlorid, in der sterilen Lösung mit einem Anteil von 0,01 bis 1,0 Gew.-%, vorzugsweise von 0,05 bis 0,5 Gew.-%, insbesondere von 0,1 Gew.-% enthalten ist.

3. Applikationsspritze (1) nach einem der vorherigen Ansprüche, **dadurch gekennzeichnet, dass** die den medizinischen Wirkstoff enthaltende, sterile Lösung eine Oxybutynin-Hydrochlorid-haltige NaCl-Lösung ist.

4. Applikationsspritze nach (1) Anspruch 3, **dadurch gekennzeichnet, dass** die den medizinischen Wirkstoff enthaltende, sterile Lösung eine Oxybutynin-Hydrochlorid-haltige, isotonische NaCl-Lösung ist.

5. Applikationsspritze (1) nach einem der vorhergehenden Ansprüche, **dadurch gekennzeichnet, dass** der Stufenkonus (4) 10 bis 15 zylinderförmige Abschnitte (8) mit unterschiedlichen, zu der Spitze des Stufenkonus (4) hin kleiner werdenden Durchmessern aufweist.

6. Applikationsspritze (1) nach einem der vorhergehenden Ansprüche, **dadurch gekennzeichnet, dass** der Kolben aus einem autoklavierbaren Brombutylkautschuk besteht.

7. Applikationsspritze (1) nach einem der vorhergehenden Ansprüche, **dadurch gekennzeichnet, dass** die Oxybutynin-Hydrochlorid-haltige Lösung mit einem Volumen von 1 bis 50 ml, vorzugsweise von 5 bis 25 ml, insbesondere von 10 ml, in dem Hohlraum (6) angeordnet ist.

8. Applikationsspritze (1) nach einem der vorhergehenden Ansprüche, **dadurch gekennzeichnet, dass** die Auslassöffnung (5) mit einem Verschlusselement, insbesondere einer Verschlusskappe, aus einem halogenierten Isobuten-Isopren-Kautschuk verschlossen ist.

9. Applikationsspritze (1) nach einem der vorhergehenden Ansprüche, **dadurch gekennzeichnet, dass** diese sterilisiert verpackt ist.

10. Verfahren zum Bereitstellen einer ein Medikament enthaltenden Applikationsspritze (1) für das Einbringen des Medikaments über einen Harnwegs- und/oder Blasenkatheter in die Blase eines Patienten, nach einem der vorhergehenden Ansprüche, mit folgenden Schritten:
• Bereitstellen einer sterilen, einen zylinderförmigen Mantel (2), der einseitig durch einen einstückig mit dem Mantel (2) ausgebildeten, eine Auslassöffnung (5) aufweisenden Spritzendeckel (3) verschlossenen ist und aus einem Cycloolefin-Copolymer (COC) besteht, und einen Kolben aus einem halogenierten Isobuten-Isopren-Kautschuk aufweisenden Applikationsspritze (1),
• Bereitstellen einer Oxybutynin-Hydrochlorid-haltigen Lösung als Medikament,
• Befüllen eines von dem zylinderförmigen Mantel (2) mit Spritzendeckel (3) und dem Kolben begrenzten Hohlraums (6) der Applikationsspritze (1) mit der Oxybutynin-Hydrochlorid-haltigen Lösung,
• Verschließen der Auslassöffnung (5) der Applikationsspritze (1) mit einem Verschlusselement aus einem Isobuten-Isopren-Kautschuk,
• Dampfdruck-Sterilisieren der mit der Oxybutynin-Hydrochlorid-haltigen Lösung befüllten, mit dem Verschlusselement verschlossenen Applikationsspritze (1) in einem Autoklav.

11. Verfahren nach Anspruch 10, **dadurch gekennzeichnet, dass** der Schritt des Bereitstellens der sterilen, den zylinderförmigen Mantel (2), der einseitig durch den einstückig mit dem Mantel (2) ausgebildeten, eine Auslassöffnung (5) aufweisenden Spritzendeckel (3) verschlossenen ist und aus einem Cycloolefin-Copolymer (COC) besteht, und einen Kolben aus einem halogenierten Isobuten-Isopren-Kautschuk aufweisenden Applikationsspritze (1) das Bereitstellen einer solchen Applikationsspritze (1) mit einer in einem einstückig mit dem Mantel (2) und dem Spritzendeckel (3) ausgebildeten Stufenkonus (4) mit wenigstens zwei, vorzugsweise wenigstens drei, zylinderförmigen Abschnitten (8) mit unterschiedlichen, zu einer Spitze des Stufenkonus (4) hin kleiner werdenden Durchmessern ausgebildeten Auslassöffnung (5) umfasst.

12. Verfahren nach einem der Ansprüche 10 oder 11, **dadurch gekennzeichnet, dass** das Dampfdruck-Sterilisieren in dem Autoklav für wenigstens 10 min, insbesondere für wenigstens 15 min, insbesondere für 20 min, bei wenigstens 115°C, insbesondere bei wenigstens 120°C, insbesondere bei 121°C durchgeführt wird.

13. Verfahren nach einem der Ansprüche 10 bis 12, **dadurch gekennzeichnet, dass** es weiterhin den Schritt des Verpackens der Dampfdruck-sterilisierten, mit der Oxybutynin-Hydrochlorid-haltigen Lösung befüllten, mit dem Verschlusselement verschlossenen Applikationsspritze (1) umfasst.

## Claims

1. An application syringe (1) containing a sterile solution with a medicinal active ingredient for introducing the solution containing the active ingredient into a patient's bladder via a urinary tract and/or bladder catheter, wherein
• the application syringe (1) has a cylindrical jacket (2) which is closed by a syringe cap (3) formed integrally with the jacket (2) and having an outlet opening (5), and is formed from a cycloolefin copolymer (COC), and a plunger made of a halogenated isobutene-isoprene rubber, and
• the sterile solution containing the medicinal agent is arranged in a cavity (6) bounded by the cylindrical jacket (2) with syringe cap (3) and the piston,
**characterised in that** the outlet opening (5) is formed in a stepped cone (4) formed integrally with the jacket (2) and the syringe cap (3) with at least two, preferably at least three, cylindrical sections (8) with different diameters decreasing towards a tip of the stepped cone (4)
and that oxybutynin hydrochloride is contained in the sterile solution as the active medical ingredient.

2. Application syringe (1) according to claim 1, **characterised in that** the oxybutynin hydrochloride is contained in the sterile solution in a proportion of 0.01 to 1.0 wt.%, preferably 0.05 to 0.5 wt.%, in particular 0.1 wt.%.

3. Application syringe (1) according to one of the previous claims, **characterised in that** the sterile solution containing the active medical ingredient is a NaCl solution containing oxybutynin hydrochloride.

4. Application syringe according to (1) claim 3, **characterised in that** the sterile solution containing the medical active ingredient is an isotonic NaCI solution containing oxybutynin hydrochloride.

5. Application syringe (1) according to one of the preceding claims, **characterised in that** the stepped cone (4) has 10 to 15 cylindrical sections (8) with different diameters that decrease towards the tip of the stepped cone (4).

6. Application syringe (1) according to one of the preceding claims, **characterised in that** the piston consists of autoclavable bromobutyl rubber.

7. Application syringe (1) according to one of the preceding claims, **characterised in that** the solution containing oxybutynin hydrochloride with a volume of 1 to 50 ml, preferably 5 to 25 ml, in particular 10 ml, is arranged in the cavity (6).

8. Application syringe (1) according to one of the preceding claims, **characterised in that** the outlet opening (5) is closed with a closure element, in particular a closure cap, made of halogenated isobutene-isoprene rubber.

9. Application syringe (1) according to one of the preceding claims, **characterised in that** it is packaged in a sterile manner.

10. Method for providing an application syringe (1) containing a medication for introducing the medication into the bladder of a patient via a urinary tract and/or bladder catheter, according to one of the preceding claims, with the following steps:
• providing a sterile syringe (1) containing a drug, comprising a cylindrical jacket (2) which is closed on one side by a syringe cap (3) formed integrally with the jacket (2) and having an outlet opening (5), and consists of a cycloolefin copolymer (COC), and a plunger made of a halogenated isobutene- isoprene rubber,
• providing a solution containing oxybutynin hydrochloride as a medication,
• filling a cavity (6) of the application syringe (1), bounded by the cylindrical jacket (2) with syringe cap (3) and the plunger, with the solution containing oxybutynin hydrochloride,
• sealing the outlet opening (5) of the application syringe (1) with a sealing element made of isobutene-isoprene rubber,
• steam pressure sterilisation of the application syringe (1) filled with the solution containing oxybutynin hydrochloride and sealed with the closure element in an autoclave.

11. Method according to claim 10, **characterised in that** the step of providing the sterile application syringe (1) comprising a cylindrical jacket (2) which is closed on one side by the syringe cap (3) formed integrally with the jacket (2) and having an outlet opening (5) and consisting of a cycloolefin copolymer (COC), and a piston made of a halogenated isobutene-isoprene rubber, comprises providing such an application syringe (1) with a stepped cone (4) formed in a one-piece (2) and the syringe cap (3), with at least two, preferably at least three, cylindrical sections (8) with different diameters that become smaller towards a tip of the stepped cone (4).

12. Method according to one of claims 10 or 11, **characterised in that** the steam pressure sterilisation in the autoclave is carried out for at least 10 minutes, in particular for at least 15 minutes, in particular for 20 minutes, at at least 115°C, in particular at at least 120°C, in particular at 121°C.

13. Method according to one of claims 10 to 12, **characterised in that** it further comprises the step of packaging the steam pressure sterilised application syringe (1) filled with the oxybutynin hydrochloride-containing solution and closed with the closure element.

## Revendications

1. Seringue d'application (1) contenant une solution stérile avec un principe actif médicinal pour introduire la solution contenant le principe actif dans la vessie d'un patient via un cathéter urinaire et/ou vésical, dans laquelle
• la seringue d'application (1) comporte une enveloppe cylindrique (2) qui est fermée par un capuchon de seringue (3) formé d'un seul tenant avec l'enveloppe (2) et comportant une ouverture de sortie (5), et qui est formée d'un copolymère de cyclooléfine (COC), et un piston en caoutchouc isobutène-isoprène halogéné, et
• la solution stérile contenant l'agent médicinal est disposée dans une cavité (6) délimitée par l'enveloppe cylindrique (2) avec le capuchon de seringue (3) et le piston,
**caractérisée en ce que** l'ouverture de sortie (5) est formée dans un cône étagé (4) formé d'un seul tenant avec l'enveloppe (2) et le capuchon de seringue (3) avec au moins deux, de préférence au moins trois, sections cylindriques (8) de diamètres différents diminuant vers une pointe du cône étagé (4)
et **en ce que** le chlorhydrate d'oxybutynine est contenu dans la solution stérile en tant que principe actif médical.

2. Seringue d'application (1) selon la revendication 1, **caractérisée en ce que** le chlorhydrate d'oxybutynine est contenu dans la solution stérile dans une proportion de 0,01 à 1,0 % en poids, de préférence de 0,05 à 0,5 % en poids, en particulier de 0,1 % en poids.

3. Seringue d'application (1) selon l'une des revendications précédentes, **caractérisée en ce que** la solution stérile contenant le principe actif médical est une solution de NaCl contenant du chlorhydrate d'oxybutynine.

4. Seringue d'application selon la revendication 3, **caractérisée en ce que** la solution stérile contenant le principe actif médical est une solution isotonique de NaCl contenant du chlorhydrate d'oxybutynine.

5. Seringue d'application (1) selon l'une des revendications précédentes, **caractérisée en ce que** le cône étagé (4) comporte 10 à 15 sections cylindriques (8) de diamètres différents qui diminuent vers la pointe du cône étagé (4).

6. Seringue d'application (1) selon l'une des revendications précédentes, **caractérisée en ce que** le piston est constitué de caoutchouc bromobutyle autoclavable.

7. Seringue d'application (1) selon l'une des revendications précédentes, **caractérisée en ce que** la solution contenant du chlorhydrate d'oxybutynine d'un volume de 1 à 50 ml, de préférence de 5 à 25 ml, en particulier de 10 ml, est disposée dans la cavité (6).

8. Seringue d'application (1) selon l'une des revendications précédentes, **caractérisée en ce que** l'ouverture de sortie (5) est fermée par un élément de fermeture, en particulier un capuchon de fermeture, en caoutchouc isobutène-isoprène halogéné.

9. Seringue d'application (1) selon l'une des revendications précédentes, **caractérisée en ce qu'**elle est conditionnée de manière stérile.

10. Procédé pour fournir une seringue d'application (1) contenant un médicament pour introduire le médicament dans la vessie d'un patient via un cathéter urinaire et/ou vésical, selon l'une des revendications précédentes, comprenant les étapes suivantes :
• fournir une seringue stérile (1) contenant un médicament, comprenant une enveloppe cylindrique (2) qui est fermée d'un côté par un capuchon de seringue (3) formé d'un seul tenant avec l'enveloppe (2) et comportant une ouverture de sortie (5), et qui est constituée d'un copolymère de cyclooléfine (COC), et un piston en caoutchouc isobutène-isoprène halogéné,
• fournir une solution contenant du chlorhydrate d'oxybutynine comme médicament,
• remplir une cavité (6) de la seringue d'application (1), délimitée par l'enveloppe cylindrique (2) avec le capuchon de seringue (3) et le piston, avec la solution contenant du chlorhydrate d'oxybutynine,
• fermer l'ouverture de sortie (5) de la seringue d'application (1) avec un élément d'étanchéité en caoutchouc isobutène-isoprène,
• stérilisation à la vapeur sous pression de la seringue d'application (1) remplie de la solution d' e contenant du chlorhydrate d'oxybutynine et scellée avec l'élément de fermeture dans un autoclave.

11. Procédé selon la revendication 10, **caractérisé en ce que** l'étape consistant à fournir la seringue d'application stérile (1) comprenant une enveloppe cylindrique (2) qui est fermée d'un côté par le capuchon de seringue (3) formé d'un seul tenant avec l'enveloppe (2) et comportant une ouverture de sortie (5) et constituée d'un copolymère de cyclooléfine (COC), et d'un piston en caoutchouc isobutène-isoprène halogéné, comprend la fourniture d'une telle seringue d'application (1) avec un cône étagé (4) formé d'une seule pièce (2) et le capuchon de seringue (3), avec au moins deux, de préférence au moins trois, sections cylindriques (8) de diamètres différents qui deviennent plus petites vers une pointe du cône étagé (4).

12. Procédé selon l'une des revendications 10 ou 11, **caractérisé en ce que** la stérilisation à la vapeur sous pression dans l'autoclave est effectuée pendant au moins 10 minutes, en particulier pendant au moins 15 minutes, en particulier pendant 20 minutes, à au moins 115°C, en particulier à au moins 120°C, en particulier à 121°C.

13. Procédé selon l'une des revendications 10 à 12, **caractérisé en ce qu'**il comprend en outre l'étape consistant à emballer la seringue d'application (1) stérilisée à la vapeur sous pression, remplie de la solution contenant du chlorhydrate d'oxybutynine et fermée avec l'élément de fermeture.
